# EUROPEAN PATENT APPLICATION

(11) **EP 3 632 445 A1**
(43) Date of publication of application: **08.04.2020**
(21) Application number: 18198631.6
(22) Date of filing: 04.10.2018
(51) Int. Cl.: A61K 31/568, A61K 45/06, A61P 35/00, A61K 31/337, A61K 31/519, A61K 31/565, A61K 31/138, A61K 31/277, A61K 31/4166, A61K 31/5685, A61K 31/58

(54) **COMBINATIONS WITH A C-19 STEROID FOR TREATING CANCERS**

(71) Applicant: Curadis GmbH, 90409 Nürnberg (DE)
(72) Inventor: UNTEREGGER, Gerhard, 66424 Homburg/Saar (DE); SCHÖNFELD, Wolfgang, 2100 Korneuburg (AT)
(74) Representative: Prüfer & Partner mbB Patentanwälte · Rechtsanwälte

(57) **Abstract**

The present invention relates to the field of medicine, particularly to novel uses of C-19 steroid compounds, more particularly to C-19 steroids having an androsten-3-one structure with specific structural configurations, in particular at the 4- and/or 17-position, for inhibition of cancer cell growth in combination with another anticancer agent or as second or third line treatment. The present invention particularly relates to selected C-19 steroids inhibiting the proliferation of tumor cells expressing an androgen receptor and show only limited sensitivity to other cancer agents such as aromatase inhibitors, androgen receptor antagonists, androgen synthesis inhibitors, chemotherapeutics as well as CDK4/CDK6 inhibitors.

## Description

### Field of the invention

The present invention relates to the field of medicine, particularly to novel uses of C-19 steroid compounds, more particularly to C-19 steroids having an androsten-3-one structure with specific structural configurations, in particular at the 4- and/or 17-position, for inhibition of cancer cell growth in combination with another anticancer agent or as second or third line treatment. The present invention particularly relates to selected C-19 steroids inhibiting the proliferation of tumor cells expressing an androgen receptor and show only limited sensitivity to other cancer agents such as aromatase inhibitors, androgen receptor antagonists, androgen synthesis inhibitors, chemotherapeutics as well as CDK4/CDK6 inhibitors.

### Description of the background art

The therapy of cancer is still a challenge for physicians and patients. Numerous approaches are used in the clinical setting based on (i) radiation, i.e. killing of tumor by radioactive materials or high energy beams, (ii) surgery, i.e. removal of tumor and its metastases, (iii) chemotherapy, i.e. treatment with compounds which kill tumor cells or inhibit their growth, (iv) immune modifiers, e.g. stimulation of the body's own host defence to kill tumors by certain populations of lymphocytes, or (v) targeted therapies, e.g. specifically inhibiting e.g. by antibodies a certain biochemical pathways without which a tumor cell cannot grow and may die.

Commonly, the above-described approaches for the treatment of cancers are used in combination. For instance, surgery may be followed by chemotherapy or targeted therapy, or combination of various molecules to realize a desirable effect - highest efficacy and acceptable toxicity, e.g. chemotherapy protocols using two to four different compounds depending on the specific tumor entity.

In the context of breast cancer, cytostatic acting drugs are frequently used in combination with hormone influencing therapies. For example, Tamoxifen, an estrogen receptor blocker is combined with cyclophosphamide (Cytoxan), doxorubicin (Adriamycin), UFT (uracil and tegafur), (so-called "CAUT regimen"). Furthermore, chemotherapy combinations comprise Adriamycin and Cytoxan (AC), Adriamycin and Taxotere (AT), Cytoxan, methotrexate, and fluorouracil (CMF), fluorouracil, Adriamycin, and Cytoxan (FAC), Cytoxan, Adriamycin, and fluorouracil (CAF). Furthermore, also newer drugs such as the PDK4/6 inhibitors (e.g. Palbociclib (Ibrance)) are used in estrogen receptor positive tumor patients with aromatase inhibitors (letrozole/anastrozole). Frequently, advanced breast cancer is associated with multidrug resistance, which occurs in the vast majority of patients treated with chemotherapy.

Likewise, combination therapies are recommended for the treatment of advanced prostate cancer, *inter alia* to overcome therapy resistance. Indeed, it is an exceptional challenge to combat therapy resistance in the management of castration-resistant prostate cancer (CRPC) due to primary or acquired resistance. This may be caused by the remarkable molecular heterogeneity and tumor adaptability in advanced prostate cancer longing for an optimization of treatment strategies. The fundamental principle of prostate cancer therapy is based on the inhibition of androgen synthesis and/or androgen receptor antagonism. Additionally, protocols are used combining this therapy regimens with chemotherapeutic agents (e.g. docetaxel or corticoids).

However, these therapies may not show any therapeutic effect due to primary insensitivity or primary or acquired resistance towards the anticancer agents. Furthermore, the administration of the above-mentioned combination therapies may be accompanied with several severe or less severe side effects. Hence, there is a need for a novel approach to influence sensitivity or even resistance of cancer cells towards known or future anti-cancer agents. Furthermore, there is a need for a novel approach in treating cancers, the approach being associated with less side effects.

Recently, the activity of 4-hydroxytestosterone (4-OHT) on tumor cells and angiogenesis has been described (EP 2 564 900 A1, EP 2 060 300 A1, EP 2 018 169 A1, and EP 3 040 075 A1). 4-OHT is a molecule closely related to testosterone and is targeting the androgen receptor. In line with the general desire to apply combinations of treatments, and as accordingly envisaged in these patent documents referring generally to combinations, in the experimental settings of these documents 4-OHT was used as a single compound.

Considering the problems of conventional monotherapies and combination therapies mentioned above, it is therefore an object of the present invention to provide an improved anti-cancer therapy. A particular problem lies in reducing the side-effects of cancer therapy, for example by enhancing the response of a patient to smaller strength/dosage of an anti-cancer therapy. Furthermore, it is an objective of the present invention to provide an improved anti-cancer therapy that is particularly useful in enhancing a response of a patient who is less responsive, insensitive or even resistant to an anti-cancer therapy.

In solving the object, the present invention provides combinations using a specifically selected C19 steroid compounds for particular applications as set forth in the appended claims.

### Summary of the Invention

The present invention provides the following aspects, subject-matters and preferred embodiments, which respectively taken alone or in combination, further contribute to solving the object of the present invention:
1. A combination for use in the therapy of cancer comprising:
   (i) a first active compound being selected from compounds having the following formula I: 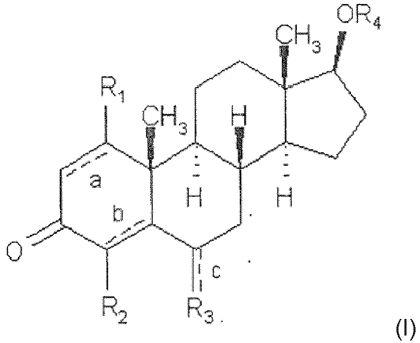
      wherein a, b and c respectively denote, independently from each other, a single bond or a double bond, with the proviso that at least one of a, b and c represents a double bond, and with the proviso that if a is single bond and b is double bond R₂ is not H;
      R₁ is hydrogen or C₁ to C₆ alkyl;
      R₂ is OR5 or hydrogen, wherein R₅ is hydrogen or C₁ to C₆ straight chain or branched alkyl; R₃ is, in case of c being a single bond, hydrogen or C₁ to C₆ alkyl, or in case of c being a double bond, CHR₅, wherein R₅ is the same as defined before;
      R₄ is hydrogen, C₁ to C₆ alkyl, phenyl unsubstituted or substituted by C₁ to C₆ alkyl, COR₆ acyl group, wherein R₆ is hydrogen; C₁ to C₁₂ straight chain or branched alkyl; phenyl or benzoyl respectively unsubstituted or substituted by C₁ to C₆ alkyl, or any group leading to hydroxyl upon biological metabolization or chemical deprotection; and salts thereof;
   (ii) a second active anti-cancer compound different from compound (i),
   and wherein compound (ii) is used under one or more of the following conditions:
   (a) for a patient to be sensitized for said anti-cancer compound (ii), the patient being insensitive or resistant against therapy by said anti-cancer compound;
   (b) at a dose and/or a dosage regimen of a known and conventionally used anti-cancer agent as second compound (ii), lower than the dose and/or dosage regimen conventionally used or admitted for said compound (ii) in the therapy of the same cancer;
   (c) for a patient who, due to side effects caused by the second active anti-cancer compound (ii), would without the first active compound (i) be determined for treatment by an add-on therapy to counteract the side effects;
   (d) for a patient who cannot be treated with said second active anti-cancer compound (ii) at a dose and/or a dosage regimen of a known and conventionally used anti-cancer agent as second compound (ii) conventionally used or admitted for said compound (ii) in the therapy of the same cancer due to side effects caused by said anti-cancer compound (ii), or for a patient who cannot be treated with said second active anti-cancer compound (ii) due to side effects caused by said anti-cancer compound (ii) at all.
2. A compound (i) of formula (I) as set forth under item 1 for use in the treatment of a patient suffering from cancer, who previously has been treated with at least one other anti-cancer compound and has failed with such previous treatment.
3. The compound (i) for use according to item 2, wherein the cancer is a breast cancer and the at least one other anti-cancer compound that has failed is an anti-estrogen, particularly an aromatase inhibitor, and/or an estrogen receptor modulator, particularly tamoxifen and/or an estrogen receptor antagonist, particularly fulvestrant.
4. The compound (i) for use according to item 2, wherein the cancer is prostate cancer and the at least one other anti-cancer compound that has failed is a GnRH agonist and/or Docetaxel, an androgen synthesis inhibitor, particularly abiraterone, or bicalutamide and/or androgen receptor antagonist particularly enzalutamide.
5. The combination or compound (i) for use according to any of the preceding items, wherein compound (i) is defined by a and c being a single bond, b being a double bond, and R₂ being OR5, OR5 being as defined in claim 1.
6. The combination or compound (i) for use according to any of the preceding items, wherein compound (i) is 4-OHT or a salt or an analogue thereof.
7. The combination or compound (i) for use according to any of the preceding items, wherein the second active anti-cancer compound (ii) is selected from the group consisting of a CDK4/CDK inhibitors; an aromatase inhibitor; an estrogen antagonist; an estrogen receptor modulator, a chemotherapeutic drug; an HER2/neu receptor antagonist; an androgen receptor inhibitor; an androgen synthesis inhibitor; an androgen synthesis antagonist; a GnRH receptor antagonist; a GnRH agonist; a microtubule inhibitor; an anthracenedione antineoplastic agent; steroids, specific antibody therapies; immune therapeutics.
8. The combination or compound (i) for use according to any of the preceding items, wherein the second active anti-cancer compound (ii) is selected from the group consisting of Palbociclib, Ribociclib, Abemaciclib; Exemestan, Anastrozole, Letrozole; Fulvestrant; Endoxifen, Tamoxifen, Raloxifene, Ospemifene, Toremifene, Docetaxel, Abraxane, Doxorubicin, or Cyclophsophamide; Trastuzumb; Bicalutamide, Flutamide, Nilutamide; Enzalutamide, Abiraterone, Apalutamide; Goserelin; Degarelix; Leuprolide; Cabazitaxel, Mitoxantrone.
9. The combination or compound (i) for use according to any of the preceding items, wherein the cancer is a cancer and/or metastasis thereof that is conventionally treated with the second active anti-cancer compound (ii) as defined in any of the preceding items.
10. The combination or compound (i) for use according to any of the preceding items, wherein the cancer is a breast cancer, an ovarian cancer, an endometrial cancer, a prostate cancer or a testis cancer.
11. The combination or compound (i) for use according to any of the preceding items, wherein the cancer is a breast cancer or a prostate cancer.
12. The combination or compound (i) for use according to any of the preceding items, wherein the cancer is an advanced breast cancer or an advanced prostate cancer.
13. The combination or compound (i) for use according to any of the preceding items, wherein the cancer is AR-positive.
14. The combination or compound (i) for use according to any of the preceding items, wherein the cancer is AR-positive with an AR-expression of at least 1%.
15. The combination for use according to any of the preceding items, wherein the first active compound (i) and the second active anti-cancer compound (ii) are administered to a patient simultaneously or in the order of firstly using or releasing the first active compound (i) and subsequently using or releasing the second active anti-cancer compound (ii), respectively, optionally in common or in separate dosage forms.
16. A combination comprising:
   (i) a compound (i) of formula (I) as set forth in any of the preceding items, and
   (ii) an anti-cancer compound different from compound (i),
      wherein said anti-cancer compound is selected from the group consisting of:
      a CDK4/CDK inhibitor, preferably Palbociclib, Ribociclib, or Abemaciclib;
      Exemestan;
      Anastrozole;
      Letrozole;
      Fulvestrant;
      an estrogen receptor modulator, particularly Endoxifen, Tamoxifen, Raloxifene, Ospemifene, Toremifene;
      a chemotherapeutic drug, particularly Docetaxel, Abraxane, Doxorubicin, or Cyclophsophamide;
      Trastuzumb;
      Nilutamide;
      an androgen synthesis inhibitor, particularly Enzalutamide, Abiraterone, Apalutamide;
      an androgen synthesis antagonist, particularly Goserelin;
      a GnRH receptor antagonist, particularly Degarelix;
      a GnRH agonist, particularly Leuprolide;
      a microtubule inhibitor, particularly Cabazitaxel;
      an anthracenedione antineoplastic agent, particularly Mitoxantrone;
      immunotherapeutically active substance including specific tumor antibody and/or immune therapeutic agent, particularly monoclonal antibodies to treat cancer, chimeric antigen receptor (CAR) T-cell therapy, immune checkpoint inhibitors to treat cancer, cancer vaccines and/or non-specific cancer immunotherapies and adjuvants, particularly cytokines including interleukins and interferons.
17. The combination according to item 16, wherein the anti-cancer agent is selected from the group consisting of Palbociclib, Ribociclib, Abemaciclib, Exemestan, Anastrozole, Letrozole, Fulvestrant, Endoxifen, Tamoxifen, Raloxifene, Ospemifene, Toremifene, Docetaxel, Abraxane, Doxorubicin, Cyclophsophamide, Trastuzumb, Nilutamide, Enzalutamide, Abiraterone, Apalutamide, Goserelin, Degarelix, Leuprolide, Cabazitaxel, Mitoxantrone.
18. A combination according to any of items 16 to 17 for use in the therapy of cancer.
19. The combination for use according to item 18, wherein the cancer is AR-positive.
20. The combination for use according to any of items 18 or 19, wherein the cancer is AR-positive with an AR-expression of at least 1%.
21. The combination for use according to any of items 18 to 20, wherein the cancer is a cancer and/or metastasis thereof that is conventionally treated with the second active anti-cancer compound (ii) as defined in any of the preceding items.
22. The combination for use according to any of items 18 to 21, wherein the cancer is breast cancer or prostate cancer.
23. The combination for use according to any of items 18 to 22, wherein the cancer is an advanced breast cancer or an advanced prostate cancer.
24. The combination according to item 16 or 17 or the combination for use according to any of items 18 to 23, wherein the first active compound (i) and the second active anti-cancer compound (ii) are comprised in a common or in separate dosage forms.
25. The combination according to any one of the preceding items, wherein the combination is arranged in a package together with instructions providing descriptions in relation to any one of conditions (a) to (d) or any items 2 to 4, in particular descriptions relating to specific dose and/or dosage regimen.
26. A pharmaceutical formulation for use according to any of the preceding claims comprising the first active compound (i), and optionally the second active anti-cancer compound (ii).
27. The pharmaceutical formulation for use according to item 26, wherein the formulation is for topical, transdermal, parenteral, intranasal, oral, rectal, intraocular administration and/or for instillation, preferably wherein the formulation is for topical administration.

### Description of the drawings

- Fig. 1: Proliferation assay of T-47D cells with Palbociclib as single drug and in combination with 4-OHT
- Fig. 2: Proliferation assay of T-47D cells with Fulvestrant and Exemestan as single drugs and in combination with 4-OHT
- Fig. 3A: Proliferation assay of T-47D cells with Docetaxel as single drug and in combination with 4-OHT
- Fig. 3B: Proliferation assay of ZR-75-1N cells with Docetaxel as single drug and in combination with 4-OHT
- Fig. 4A: Proliferation assay of LNCaP cells with Bicalutamide and Enzalutamide as single drugs and in combination with 4-OHT
- Fig. 4B: Proliferation assay of VCaP cells with Bicalutamide and Enzalutamide as single drugs and in combination with 4-OHT
- Fig. 5: Proliferation assay of LNCaP cells with Abiraterone as single drug and in combination with 4-OHT

### Detailed description of the invention

As used herein, the term "add-on therapy" refers to a therapy other than with compound (i) that is prescribed, determined, recommended or necessary for ameliorating side effects and or increasing therapeutic efficacy arising with the administration or release of the second active anti-cancer compound (ii).

As used herein, the expression "advanced breast cancer" includes "locally advanced breast cancer", i.e. invasive breast cancer that has spread to areas near the breast, such as the chest wall (Stage III), as well as "metastatic breast cancer", i.e. invasive breast cancer that has spread from the breast to other parts of the body (Stage IV).

As used herein, the expression "advanced prostate cancer" includes "locally advanced prostate cancer", i.e. the cancer extends beyond the prostate and may have invaded the seminal vesicles, but cancer cells have not spread to the lymph nodes (Stage III), as well as "metastatic prostate cancer", i.e. the cancer may have invaded the bladder, rectum or nearby structures beyond the seminal vesicles and may have spread to the lymph nodes, bone or to other parts of the body (Stage IV).

As used herein, the term "combination" refers to a therapy that combines more than one method of therapy, i.e. is not a monotherapy only. The more than one therapies, for instance two agents, may be administered concomitantly or sequentially, optionally in the same or in separate dosage or administration forms.

As used herein, the expression a "conventional dose and/or dosage regimen of the second active anti-cancer compound (ii)" or a "dose and/or dosage regimen of the second active anti-cancer compound (ii) conventionally used" refers to a dose and/or dosage regimen of said compound (ii) as prescribed in corresponding national, multinational or supranational pharmacopeias incorporated herein by reference in the respective current edition, such as for example the Brazilian Pharmacopoeia, British Pharmacopoeia, Pharmacopoeia of the People's Republic of China (Chinese Pharmacopoeia), Czech Pharmacopoeia, Slovak Pharmacopoeia, European Pharmacopoeia, French Pharmacopoeia, German Pharmacopoeia, Hungarian Pharmacopoeia, Indian Pharmacopoeia, International Pharmacopoeia, Japanese Pharmacopoeia, Minimum Requirements for Antibiotic Products of Japan, Pharmacopoeia of the United Mexican States (Mexican Pharmacopoeia), Portuguese Pharmacopoeia, Swiss Pharmacopoeia, United States Pharmacopeia, Royal Spanish Pharmacopoeia, the German register for pharmaceutical drugs "Red List" ("Rote Liste"), the "Orange Book" of the US Food and Drug Administration (FDA) and/or the website of the FDA https://www.accessdata.fda.gov/scripts/cder/daf/index.cfm including the search tool and prescribing information available therewith, in particular as prescribed by the respective current edition or version of https://www.accessdata.fda.gov/scripts/cder/daf/index.cfm, United States Pharmacopeia, the German register for pharmaceutical drugs "Red List" and/or European Pharmacopoeia, and especially as prescribed in the prescribing information available on https://www.accessdata.fda.gov/scripts/cder/daf/index.cfm as currently available.

As used herein, the expression a "dose and/or a dosage regimen of a known and conventionally used anti-cancer agent as second compound (ii) lower than the dose and/or dosage regimen conventionally used or admitted for said compund (ii) in the therapy of the same cancer" refers either to a lower or reduced dose of said second compound (ii) by reference to the above-mentioned descriptions in national or regional pharmacopeiae, for example 95% or less than 95%, 90% or less than 90%, 85% or less than 85%, 80% or less than 80%, 75% or less than 75%, 70% or less than 70%, 65% or less than 65%, 60% or less than 60%, 55% or less than 55%, 50% or less than 50%, 45% or less than 45%, 40% or less than 40%, 35% or less than 35%, 30% or less than 30%, 25% or less than 25%, 20% or less than 20%, 15% or less than 15%, or 10% or less than 10% of the dose conventionally used, or to a lower or reduced dosage regimen of said second compound (ii), for example every second day or less frequent instead of once a day, one time or two times a day instead of three times a day of a conventional dose; or one time, two times or three times a day instead of four times a day of a conventional dose.

As used herein, the term "resistant" refers to a cancer sample or a mammal, particularly to a human patient, almost not responding or not responding at all to a therapy. As used herein, a "non-responder" is a mammal, particularly a human patient showing resistance to a therapy.

As used herein, the term "sensitizing" or "to be sensitized" refers to an increased sensitivity or reduced resistance of a cancer sample or a mammal, particularly a human patient, responding to a therapy. An increased sensitivity or a reduced sensitivity to a therapy is measured according to a known method in the art for the particular therapy and methods described herein below, including, but not limited to, cell proliferative assays and cell death assays. The sensitivity or resistance may also be measured in animals by measuring the tumor size reduction over a period of time.

A sensitizer sensitizes response to an anti-cancer agent if the increase in therapy sensitivity or the reduction in resistance is 25% or more, for example, 30%, 40%, 50%, 60%, 70%, 80%, or more, to 2-fold, 3-fold, 4-fold, or more, compared to therapy sensitivity or resistance in the absence of such a sensitizer. The determination of sensitivity or resistance to a therapy is routine in the art and within the skill of an ordinarily skilled clinician.

As used herein, the term "synergy" refers to an effect achieved by simultaneous therapy of cancer cells with the compound of formula I and the anti-cancer agent. Synergy or also called "synergistic effect" can be calculated as known in the art. For instance, synergy (SE) can be calculated as a ratio of therapy outcome _{anti-cancer compound (ii)alone}/therapy outcome _{combination} (SE₁). For example, the therapy outcome may be the absorbance at 450 nm as % of solvent control (A), if the assay used for determining the sensitivity to a therapy is an proliferation assay. Accordingly, the synergistic effect (SE) is then calculated as A_{anti-cancer compound (ii) alone}/A _{combination} (SE₁).

As used herein, the term "therapy" means prevention, prophylaxis, and treatment of a specified disease in a mammal, i.e. in a human or an animal, in particular a human patient.

As used herein, a "compound (i) of formula I" means a compound having the following structure: 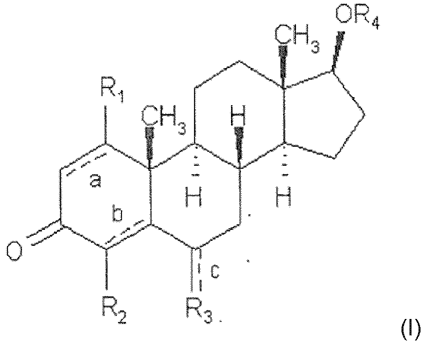 wherein a, b and c respectively denote, independently from each other, a single bond or a double bond, with the proviso that at least one of a, b and c represents a double bond, and with the proviso that if a is single bond and b is double bond R₂ is not H; R₁ is hydrogen or C₁ to C₆ alkyl; R₂ is OR₅ or hydrogen, wherein R₅ is hydrogen or C₁ to C₆ straight chain or branched alkyl; R₃ is, in case of c being a single bond, hydrogen or C₁ to C₆ alkyl, or in case of c being a double bond, CHR₅, wherein R₅ is the same as defined before; R₄ is hydrogen, C₁ to C₆ alkyl, phenyl unsubstituted or substituted by C₁ to C₆ alkyl, COR₆ acyl group, wherein R₆ is hydrogen; C₁ to C₁₂ straight chain or branched alkyl; phenyl or benzoyl respectively unsubstituted or substituted by C₁ to C₆ alkyl, or any group leading to hydroxyl upon biological metabolization or chemical deprotection; and salts thereof.

As used herein, the term "4-OHT" means 4-hydroxytestosterone. As known, 4-OHT has the following structural formula:

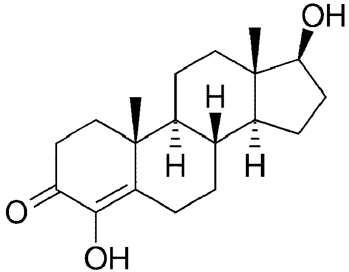

As used herein, the term "an analogue thereof", i.e. an analogue of 4-OHT, means an analogue compound in which the 4-OH group and/or the 17ß-OH group, in particular the 17ß-OH group, is/are modified by a modification group which is capable of leading upon biological metabolization to the respective OH-group, particularly wherein the modification group together with the respective OH-group forms an ester, an ether, an acetale, a carbonate, a carbamate, a phosphate, a phosphonate, a ketal, a sulfate, or a sulfonate, preferably an ester and in particular a 17-hydroxy ester of 4-OHT. The ester may be formed by modification with an acyl group, such as by forming an ester defined by a straight chain or branched alkyl group, an aryl group, an aralkyl group, for example an unsubstituted or substituted C₁ to C₁₂ alkyl group and specifically a C₁ to C₆ alkyl group.

As used herein, the term "salts thereof" or "a salt thereof", i.e. salts of the compound of formula (I) or of 4-OHT, means any salt partner of that compound or of 4-OHT. The salt is a typically pharmaceutically acceptable salt former, such as sodium salt, potassium salt, calcium salt, magnesium salt, or the like.

Surprisingly and distinct from prior investigations of related compounds to inhibition of hormone-related tumor cell growth and metastasis formation using said compound alone, it was found by the inventors that the compound 4-OHT or salts or analogues thereof have a sensitizing effect on tumor cells to other anti-cancer agents (compound ii) allowing a variety of useful applications, for instance a reduction in strength of said other anti-cancer agent. Furthermore, it was surprisingly found that said compounds have a re-sensitization effect on therapy resistant tumors to other anti-cancer agents. Hence, the finding of this (re-)sensitizing effect identifies a new clinical situation, namely one in which the strength (e.g. dosage) of another anti-cancer agents may be reduced.

Therefore, a combination comprising (i) a first active compound, the compound being 4-OHT or a salt or an analogue thereof, and (ii) a second active anti-cancer compound different from compound (i) may be used under one or more of the following conditions:
(a) for a patient to be sensitized for said anti-cancer agent, the patient being insensitive or resistant against therapy by said anti-cancer agent;
(b) at a dose and/or a dosage regimen of a known and conventionally used anti-cancer agent as second compound (ii), lower than the dose and/or dosage regimen conventionally used or admitted for said compound (ii) in the therapy of the same cancer;
(c) for a patient who, due to side effects caused by the second active anti-cancer compound (ii), would without the first active compound (i) be determined for treatment by an add-on therapy to counteract the side effects;
(d) for a patient who cannot be treated with said second active anti-cancer compound (ii) at a dose and/or a dosage regimen of a known and conventionally used anti-cancer agent as second compound (ii) conventionally used or admitted for said compound (ii) in the therapy of the same cancer due to side effects caused by said anti-cancer compound (ii) or for a patient who cannot be treated with said second active anti-cancer compound (ii) due to side effects caused by said anti-cancer compound (ii) at all.

In another aspect of the present invention, a compound (i) of formula (I) as defined above, particularly in the items, is provided for use in the treatment of a patient suffering from cancer, who previously has been treated with at least one other anti-cancer compound and has failed with such previous treatment. The other anti-cancer compound may be any second anti-cancer compound (ii) defined above given. Furthermore, the cancer may be any cancer mentioned above. Such, the present invention provides a compound (i) of formula (I) for use as second line or third line treatment, i.e. for cancers, which are therapy resistant against conventional cancer treatment. In this aspect of the present invention, the cancer is a breast cancer and the at least one other anti-cancer compound that has failed is an aromatase inhibitor and/or tamoxifen. Also falling under this aspect of the present invention, the cancer is prostate cancer and the at least one other anti-cancer compound that has failed is a GnRH agonist and/or Docetaxel and/or an androgen synthesis inhibitor. Particularly, the androgen synthesis inhibitor may be Enzalutamide or Abiraterone or, in particular, Finasteride and/or Dutasteride.

The patient is a mammal as defined above, particularly a human. According to the use specifications above, defined by one or more of (a) to (d) or defined by a second or third line treatment, a sub-set of patients is determined. Notably, patients suffering from cancer are under strict surveillance of a physician who will select the appropriate medication and will decide on the dosage and average duration of treatment after a thorough examination of the patient. During treatment, the physician will also diagnose side effects of a treatment or its failure and will then change the therapy or apply other therapeutic means, e.g. add-on therapies.

As evident from the examples showing a sensitization of cancer cell lines upon treatment with 4-OHT to a second anti-cancer compound (ii) as well as a synergistic effect, which is even enhanced in lower doses of the second anti-cancer compound (ii), the inventive concept of the present invention allows its application under conditions (a) to (d) above. Furthermore, it is evident that the inventive concept is effective and applicable for a variety of second anti-cancer compounds (ii), which respectively are representative for different mechanisms of action or categories.

When applied in accordance with any of the afore-mentioned condition(s), in an aspect of the present invention, the said other anti-cancer compound (ii) is selected from the group consisting of a CDK4/CDK inhibitors, preferably Palbociclib, Ribociclib, or Abemaciclib; Exemestan; Anastrozole; Letrozole; Fulvestrant; an estrogen receptor modulator, particularly Endoxifen, Tamoxifen, Raloxifene, Ospemifene, Toremifene; a chemotherapeutic drug, particularly Docetaxel, Abraxane, Doxorubicin, or Cyclophsophamide; Trastuzumb; Nilutamide; an androgen synthesis inhibitor, particularly Enzalutamide, Abiraterone, Apalutamide; an androgen synthesis antagonist, particularly Goserelin; a GnRH receptor antagonist, particularly Degarelix; a GnRH agonist, particularly Leuprolide; a microtubule inhibitor, particularly Cabazitaxel, an anthracenedione antineoplastic agent, particularly Mitoxantrone or an immunotherapy including specific antibody therapies and/or immune therapeutics, particularly monoclonal antibodies to treat cancer, chimeric antigen receptor (CAR) T-cell therapy, immune checkpoint inhibitors to treat cancer, cancer vaccines and/or non-specific cancer immunotherapies and adjuvants, particularly cytokines including interleukins and interferons without being limited thereto.

In specific combinations of compounds (i) and (ii) independent from the afore-mentioned conditions, 4-OHT or its salts or analogues is combined with a compound (ii) selected from the group of: the CDK4/CDK inhibitors is Palbociclib, Ribociclib, or Abemaciclib; the aromatase inhibitor is Exemestan, Anastrozole, or Letrozole; the estrogen antagonist is Fulvestrant; the estrogen receptor modulator is Endoxifen,Tamoxifen, Raloxifene, Ospemifene, or Toremifene; the chemotherapeutic drug is Docetaxel, Abraxane, Doxorubicin, or Cyclophsophamide; the HER2/neu receptor antagonist is Trastuzumb; the androgen receptor inhibitor is Nilutamide; the androgen synthesis inhibitor is Enzalutamide, Abiraterone, Apalutamide; the androgen synthesis antagonist is Goserelin; the GnRH receptor antagonist is Degarelix; the GnRH agonist is Leuprolide; the microtubule inhibitor is Cabazitaxel, the anthracenedione antineoplastic agent is Mitoxantrone, without being limited thereto.

In one aspect of the present invention, the target cells express AR. Preferably, AR-expression is at least 1%. The amount of AR-positive cells can be determined as known in the art, for example by immunohistochemistry and comparison of nuclear immunopositive tumor cells in the nucleus on the total of the tumor cells.

In a preferred aspect of the present invention, the cancer is selected from the group consisting of an hormone-dependent cancer, particularly breast cancer, ovarian cancer, endometrial cancer, prostate cancer, and testis cancer; a non-small cell lung cancer, a gastric adenocarcinoma, a squamous cell carcinoma of the head and neck cancer, an adenocarcinoma of the pancreas, an acute lymphoblastic leukemia, an acute myeloblastic leukemia, a Hodgkin lymphoma, a Non-Hodgkin lymphoma, a Wilms' tumor, a neuroblastoma, a soft tissue sarcoma, a bone sarcomas, a transitional cell bladder carcinoma, a thyroid carcinoma, a gastric carcinoma, a bronchogenic carcinoma, a gastric or gastroesophageal junction adenocarcinoma, and metastasis thereof.

Preferably, the cancer is a cancer and/or metastasis thereof that is conventionally treated with the second active anti-cancer compound (ii) being defined above.

More preferably, the cancer is cancer and/or metastasis thereof that has already been treated with one or more (the) second active anti-cancer compound(s) (ii) being defined above. As commonly known, cancers may develop resistance under the treatment of the above-mentioned second active anti-cancer compound (ii) involving physiological changes in the cancer cells.

In a preferred embodiment, the cancer is a breast cancer or a prostate cancer, preferably an advanced breast cancer or an advanced prostate cancer.

Particularly, the breast cancer may be selected from the group consisting of ductal carcinoma in situ (DCIS), lobular carcinoma in situ (LCIS), early breast cancer, Paget's disease, inflammatory breast cancer, locally advanced breast cancer, metastatic breast cancer or any breast cancer specifically characterized by a subset of receptors, biochemical alterations characterized by molecular typing and categorization that may be classified for example by gene expression tests including MammaTyper, MammaPrint, Oncotype DX, PAM50 ROR, genomic grade index (GGI), EndoPredict, BCI und Curebest 95GC.

Furthermore, the prostate cancer may be selected from the group consisting of locally advanced prostate cancer, castration-resistant prostate cancer, metastatic prostate cancer or any prostate cancer specifically characterized by a subset of receptors, biochemical alterations characterized by molecular typing and categorization that may be classified for example by gene expression tests.

In some embodiments, compound (i) is administered within 15 or 30 min, within 1, 2, 3, 4, 5, 6, 12, or 18 hour(s), or within 1, 2, 3, 4, or 5 days before administering the anti-cancer compound (ii). In some embodiments, compound (i) is administered concurrently with the anti-cancer compound (il). In some embodiments, compound (i) is administered within 15 or 30 min, within 1, 2, 3, 4, 5, 6, 12, or 18 hour(s), or within 1, 2, 3, 4, or 5 days after administering the anti-cancer compound (ii).

In use, in particular with reference to condition (a), the above-mentioned compound (i) may be administered to the patient in an amount suitable for sensitizing cancer cells to the other anti-cancer compound (ii). Further, the use may be determined by an appropriate application condition, such as type of patient, or type of target site or organ or pharmaceutical composition or formulation being able to transport the aforementioned activities *in vivo* to the designated final target site or organ within a patient.

For example, compound (i) may be administered topically and/or application to mucosa, e.g. in the form of an ointment, a cream, a lotion, a gel, a spray, a powder, an oil or a transdermal plaster, also comprising depot usage forms (including pellets); it may be administered parenterally, e.g. intramuscularly, or by intravenous or subcutaneous injection or infusion, or intranasal, instillation into cavities (e.g. bladder, abdomen, intestine), and/or orally, e.g., in the form of tablets, capsules, sugar or film coated tablets, liquid solutions or suspensions or rectally, e.g. in the form of suppositories, or intraocularly, e.g. in form of injection and as eye drops.

The applied amount of compound (i) for sensitizing cancer cells to the other anti-cancer compound (ii) can be suitably chosen, for example depending on the age, weight, conditions of the user and administration form; for example the dosage adopted for oral administration to adult humans may range from about 150-2000 mg per application, from 1 to 5 times daily.

The active compound (i) content of a suitable composition may be at least 0.0001 wt%, for example between 0.0001 and 20% by weight, preferably 0.6% until 10% by weight, further preferably 1 and 5% by weight, of the compound used according to the invention.

With reference to condition (b), representative and selected examples from the FDA are listed in **Table 1** (found on https://www.accessdata.fda.gov/scripts/cder/daf/index.cfm).

**Table 1 List of anti-breast and anti-prostate cancer agents (second active compound (ii)) approved by FDA until May 2018**

| **Drug Name** | **Category** | **Dosage / Dosage Regimen (selected recommended case)** | **Indication** |
|---|---|---|---|
| Palbociclib | CDK/CDK4 inhibitor | 125 mg once daily taken with food for 21 days followed by 7 days off treatment | Breast cancer |
| Ribociclib | CDK inhibitor | 600 mg orally (three 200 mg tablets) taken once daily with or without food for 21 consecutive days followed by 7 days off treatment | Breast cancer |
| Abemaciclib | CDK inhibitor | Starting dose in combination with fulvestrant: 150 mg twice daily. | Breast cancer |
| Exemestane | Aromatase inhibitor | One 25 mg tablet once daily after a meal | Breast cancer |
| Anastrozole | Aromatase inhibitor | One 1 mg tablet taken once daily | Breast cancer |
| Letrozole | Aromatase inhibitor | 2.5 mg once daily | Breast cancer |
| Fulvestrant | Estrogen receptor degrader (antagonist) | 500 mg should be administered intramuscularly into the buttocks slowly (1 - 2 minutes per injection) as two 5 mL injections, one in each buttock, on days 1, 15, 29 and once monthly thereafter. | Breast cancer |
| Docextaxel | Microtubule inhibitor (chemotherapeuti cs: cytoskeletal disrupter) | 60 mg/m² to 100 mg/m² single agent | Breast cancer; Non-Small Cell Lung Cancer Hormone Refractory Prostate Cancer (HRPC); Gastric Adenocarcinoma (GC); Squamous Cell Carcinoma of the Head and Neck Cancer (SCCHN) |
| Endoxifen | Selective estrogen receptor modulator | Three dose levels of orally administered (Z)-Endoxifen (1 mg, 2 mg, and 4 mg) were investigated once a day in 28- day cycles in cohots (used in Phase I clinical trial) | Breast cancer (Endoxifen is an active metabolite of Tamoxifen) |
| Tamoxifen | Selective estrogen receptor modulator | 20 to 40 mg daily | Breast cancer; breast cancer prevention in high-risk patients |
| Raloxifene | Selective estrogen receptor modulator | 60 mg tablet orally once daily | Osteoporosis; prevention in high-risk patients |
| Toremifene | Selective estrogen receptor modulator | 60 mg tablet orally once daily | Metastatic breast cancer in postmenopausal women with estrogen-receptor positive |
| Doxorubicin | Anthracycline topoisomerase II inhibitor | Single agent: 60 to 75 mg/m given intravenously every 21 days | Acute lymphoblastic leukemia, acute myeloblastic leukemia, Hodgkin lymphoma, Non-Hodgkin lymphoma, metastatic breast cancer, metastatic Wilms' tumor, metastatic neuroblastoma, metastatic soft tissue sarcoma, metastatic bone sarcomas, metastatic ovarian carcinoma, metastatic transitional cell bladder carcinoma, metastatic thyroid carcinoma, metastatic gastric carcinoma, metastatic bronchogenic carcinoma |
| Trastuzumb | HER2/neu receptor antagonist | 4 mg/kg over 90 minute IV infusion, then 2 mg/kg over 30 minute IV infusion weekly for 52 weeks | Breast cancer; metastatic gastric or gastroesophageal junction adenocarcinoma |
| Cyclophosphamide | Alkylating drug | Intravenous: Initial course for patients with no hematologic deficiency: 40 mg per kg to 50 mg per kg in divided doses over 2 to 5 days. | Malignant lymphomas: Hodgkin's disease, lymphocytic lymphoma, mixed-cell type lymphoma, histiocytic lymphoma, Burkitt's lymphoma; multiple myeloma, leukemias, mycosis fungoides, neuroblastoma, adenocarcinoma of ovary, retinoblastoma, breast carcinoma |
| | | Oral: Usually 1 mg per kg per day to 5 mg per kg per day for both initial and maintenance dosing. | |
| *Bicalutamide* | Androgen receptor inhibitor | In combination with an LHRH analog, one 50 mg tablet once daily | Stage D2 metastatic carcinoma of the prostate |
| Flutamide | Androgen receptor inhibitor | 250 mg every 8 hrs | Locally confined Stage B2-C and Stage D2 metastatic carcinoma of the prostate |
| Nilutamide | Androgen receptor inhibitor | 300 mg once a day for 30 days, followed thereafter by 150 mg once a day | Metastatic prostate cancer (Stage D2), as an adjunct to surgical castration. |
| *Enzalutamide* | Androgen synthesis inhibitor | 160 mg (four 40 mg capsules) administered orally once daily | Metastatic castration-resistant prostate cancer who have received prior chemotherapy containing docetaxel |
| *Abiraterone* | Androgen synthesis inhibitor (CYP17 inhibitor) | 1000 mg administered orally once daily in combination with prednisone 5 mg administered orally twice daily | Metastatic castration-resistant prostate cancer who have received prior chemotherapy containing docetaxel |
| Apalutamide | Androgen sythesis inhibitor | 240 mg (four 60 mg tablets) administered orally once daily | Non-metastatic castration-resistant prostate cancer |
| Goserelin | Androgen synthesis antagonist (CYP17 inhibitor; GnRH agonist) | 10.8 mg subcutaneously every 12 weeks into the anterior abdominal wall below the navel line | Locally confined carcinoma of the prostate; palliative treatment of advanced carcinoma of the prostate |
| Degarelix | GnRH receptor antagonist | Started with a dose of 240 mg given as two injections of 120 mg each. The starting dose is followed by maintenance doses of 80 mg administered as a single injection every 28 days | Advanced prostate cancer |
| Leuprolide | GnRH receptor agonist | 7.5 mg, 22.5 mg, 30 mg, and 45 mg injections in a kit with prefilled dual chamber syringe | Palliative treatment of advanced prostatic cancer |
| Cabazitaxel | Microtubule inhibitor | Single use vial 60 mg/1.5 mL, supplied with diluent (5.7 mL) | Hormone-refractory metastatic prostate cancer previously treated with a docetaxel-containing treatment regimen |
| Mitoxantrone | Anthracenedione antineoplastic agent. | 12 to 14 mg/m² given as a short IV infusion every 21 days (in combination with corticosteroids) | Acute myeloid leukemia; prostate cancer; multiple sclerosis |

The pharmacological composition and/or formulation of the second active anti-cancer compound (ii) may be as described in the Orange Book listed patents and the approved administration forms derivable from the pharmacopoeias listed above. Accordingly, said compounds (i) and (ii) may be comprised, in common or in separate administration forms, in pharmaceutical compositions further comprising a pharmaceutically acceptable carrier and/or excipient and/or diluent.

For topical use, the composition may be formulated by including, for example, vegetable oils and fats such as almond oil, peanut oil, olive oil, peach kernel oil, castor oil; plant extracts; ethereal oils; furthermore vegetable waxes and synthetic and animal oils; fats and waxes such as stearic acid and stearate esters, lauric acid and lauric esters, sorbitane esters, ceterayl alcohols; lecithin, lanolin alcohols, carotene, fragrances, mono- or polyhydric alcohols, urea, surfactants such as poloxamers, Tweens, emulsifiers and the like; preservatives and colorants etc.. Formulation as an oil-in-water or water-in-oil emulsion is preferred.

Solid oral forms may for example contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols, poloxamers, tocopheryl polyethylene glycol succinate (TPGS); binding agents, e.g. starches, arabic gums, gelatine, methylcellulose, carboxymethylcellulose or polyvinyl pyrrolidone; disaggregating agents, e.g. a starch, alginic acid, alginates or sodium starch glycolate; effervescing mixtures; dyestuffs, sweeteners; wetting agents, such as lecithin, polysorbates, laurylsulphates; and, in general, non-toxic and pharmacologically inactive substances used in pharmaceutical formulations. These preparations may be manufactured in known manner, for example, by means of mixing, granulating, tabletting, sugar-coating, or film-coating processes. The liquid dispersions for oral use may be e.g. syrups, emulsions and suspensions.

The syrups may contain as carrier, for example, saccharose or saccharose with glycerine and/or mannitol and/or sorbitol.

The suspensions and the emulsions may contain as carrier, for example, a natural gum, agar, sodium alginate, pectin, methylcellulose, carboxymethylcellulose, or polyvinyl alcohol, poloxamers, or TPGS.

The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

The solutions for intravenous or subcutaneous injections or infusions may contain as carrier, for example, sterile water or preferably they may be in the form of sterile, aqueous, isotonic saline solutions.

The suppositories may contain together with the active compound a pharmaceutically acceptable carrier, e.g. cocoa-butter, polyethylene glycol, a polyoxyethylene sorbitan fatty acid ester surfactant or lecithin.

If substances are admixed to promote skin penetration, their content, when using hyaluronidases, can be, for example, between 0.01 and 1% by weight, preferably 0.05 and 0.2% by weight, when using dimethylisosorbide or DMSO between 1 and 25% by weight, preferably 5 and 10% by weight, poloxamers 0.5-30%, TPGS 0.5-30%

In some embodiments, the anti-cancer compound (ii) is administered at a dose or a dosage regimen of said anti-cancer compound (ii) as conventionally used. In some embodiments, the anti-cancer compound (ii) is administered at a dose or a dosage regimen of said anti-cancer compound (ii) lower than the dose or dosage regimen conventionally admitted.

Moreover, the combination according to the present invention may comprise further active compounds different from the first active compound (i) and the second active anti-cancer compound (ii), for example a third active compound (iii), which might be another anti-cancer compound.

Frequently, cancer patients suffer from side effects caused by the therapy with the above-described anti-cancer compound (ii). These side effects include allergy, side effects on bone marrow, side effects on the liver, side effects on the kidney, heart problems, nervous system and particularly brain function side effects, anemia, appetite loss, attention, thinking, or memory problems, bleeding problems, blocked intestine or gastrointestinal obstruction, clotting problems, constipation, dehydration, dental and oral health, diarrhea, chewing or swallowing problems, dysphagia, dry mouth, xerostomia, fluid retention, edema, , fatigue, fluid around the lungs, malignant pleural effusion, fluid in the abdomen or ascites, hair loss, alopecia, hand-foot syndrome or palmar-plantar erythrodysesthesia, headaches, hearing problems, hormone deprivation symptoms in men, hypercalcemia, infection, lymphedema, menopausal symptoms in women, mental confusion, delirium, mouth sores, mucositis, nausea, vomiting, neutropenia, osteoporosis, pain, peripheral neuropathy, shortness of breath, dyspnea, skin conditions, skin reactions to targeted therapy and immunotherapy, sleeping problems, hypersomnia, somnolence syndrome, insomnia, superior vena cava syndrome, taste changes, thrombocytopenia, urinary incontinence, weight gain, weight loss.

Often, patients having these side effects are in need of a treatment with add-on therapies against said side effects. In some cases, side effects are even so severe that the therapy with said anti-cancer compound (ii) is impossible and has to be stopped. In both conditions (c) and (d), i.e. patients in need of an add-on therapy due to side effects of anti-cancer compound (ii) (condition (c), and patients not or inadequately treatable due to side effects of anti-cancer compound (ii) (condition (d), the present invention provides an approach to ameliorate side effects by lowering or reducing the dose and/or dosage regimen of the second active anti-cancer compound (ii) conventionally used.

In a preferred embodiment, the add-on therapy is administered at a dose or a dosage regimen of said add-on therapy lower than the dose or dosage regimen conventionally admitted, i.e. as prescribed in the pharmacopeias mentioned above.

The present invention is further illustrated by the description of the following examples, which are however only for illustrative purposes and shall not be understood in any limiting manner.

### Examples

### Example 1: Combination of 4-OHT with CDK4/CDK inhibitors

In a set of *in vitro* experiments, 4-OHT (20 µM) was used in combination with the CDK4/CDK inhibitor Palbociclib, currently used in therapy of breast cancer. Palbociclib was tested at various concentrations (50, 100, 200, and 400 nM) in combination with 4-OHT (20 µM).

The human breast cancer cell line T-47 D (ACC 739) was obtained from DSMZ as frozen cell pellet (Deutsche Sammlung von Mikroorganismen und Zellkulturen). Originally, these cells T-47D cell line was isolated by Keydar et al. (1979) from a pleural effusion obtained from a 54-year old female patient with an infiltrating ductal carcinoma of the breast. T-47D is expressing the ER, PR and AR but lacking HER2/neu overexpression. Thus, T-47D may represent a cell line representative for the most prevalent breast cancer subtype.

T-47D were grown routinely in RPMI medium containing 10% fetal calf serum (FCS) and antibiotics in a humidified incubator at 36.5 °C/90% humidity and 5% CO₂. Cells were maintained in cell culture bottles for less than 20 passages before the individual experiments were conducted; cell suspensions were cultured 96 well plates and exposed at various concentrations of the individual agents; incubation time varied as indicated in the respective figures. WST-1 assay was used to monitor proliferation at 450 nm wavelength.

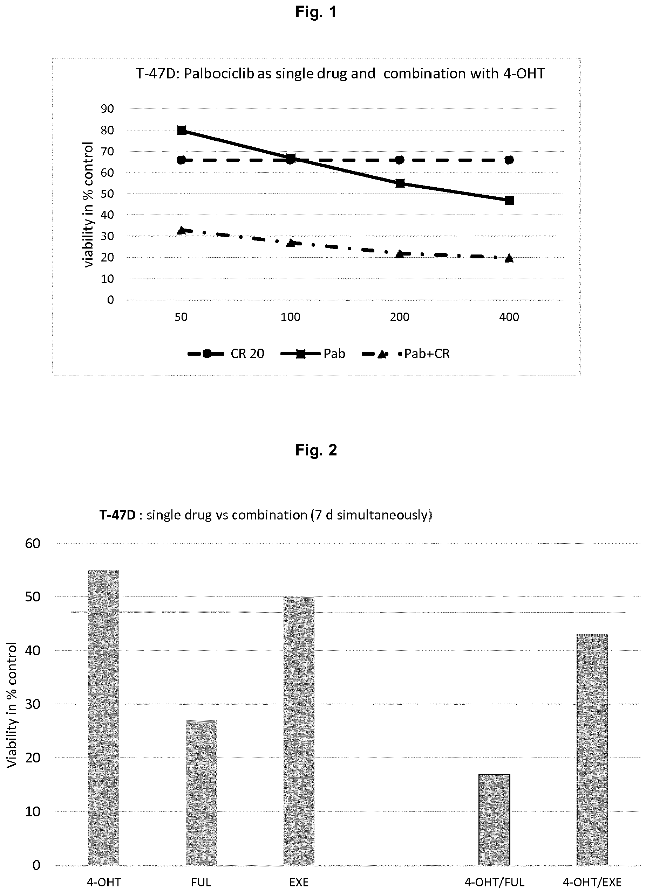

As can be seen from **Fig. 1**, combination of 4-OHT (code CR20) with Palbociclib has a synergistic effect on proliferation of T-47D cells and dose-dependently reduces cell growth as indicated by lower viability when compared to the single drugs.

### Example 2: 4-OHT in combination with an aromatase inhibitor or an estrogen antagonist

T-47 D cells described above were cultured for 7 days either in the presence of the aromatase inhibitor Exemestane (EXE), the estrogen receptor degrader Fulvestrant (FUL) or 4-OHT or a combination of 4-OHT with Fulvestrant or Exemestan with 4-OHT, respectively. Growth was as compared to control using WST-1 assay (monitoring absorption at 450 nm wavelength as indicator for cell viability compared to growth control).

As illustrated in **Fig. 2**, combination of Exemestan or Fulvestrant with 4-OHT results in a significant growth inhibition when comparing to the individual substances alone.

### Example 3: 4-OHT in combination with chemotherapeutics

In another series of experiments, the effect of the combination of 4-OHT and chemotherapeutics was assessed.

T-47D cells were grown routinely in RPMI medium containing 10% fetal calf serum (FCS) and antibiotics in a humidified incubator at 36.5 °C/90% humidity and 5% CO₂. Cells were maintained in cell culture bottles for less than 20 passages before the individual experiments were conducted; cell suspensions were cultured 96 well plates and exposed at various concentrations of Docetaxel (1, 2, 4, and 8 nM) for 5 days. WST-1 assay was used to monitor proliferation at 450 nm wavelength.

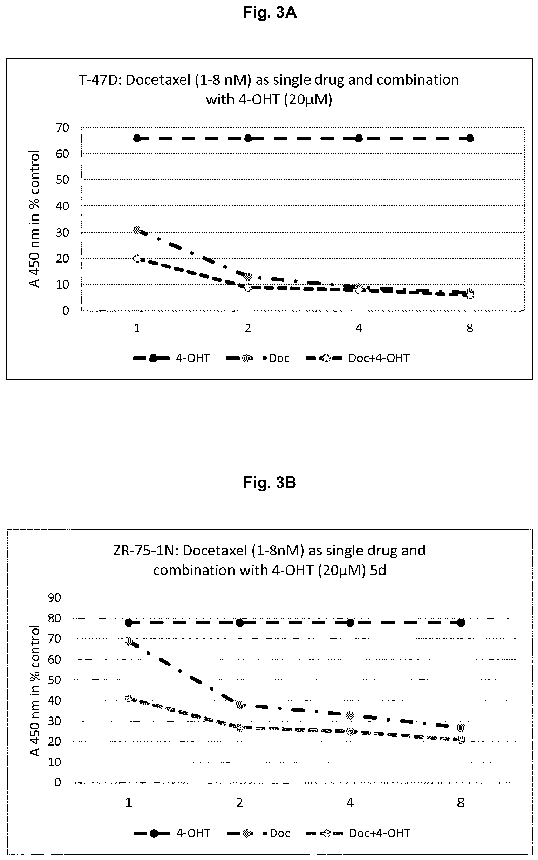

As can be seen in **Fig. 3A**, combination of 4-OHT with Docetaxel has a synergistic effect on proliferation of T-47D cells and dose-dependently reduces cell growth when comparing to the single drugs. Remarkably, the synergistic effect is highest at the lowest concentration indicating that the dose of Docetaxel given to a patient in need of an anti-cancer therapy might dramatically be reduced.

Further the human breast cancer cell line ZR-75-1N was cultured as described above and subjected to 4-OHT at a concentration of 20 µM and Docextaxel (Doc) at various concentrations (1, 2, 4, and 8 nM)) as single drugs or in combination for 5 days.

As illustrated in **Fig. 3B**, a clear effect of the combination is seen at the lowest dose, which is even visible at higher Docetaxel concentration. Results represent the average of 3 independent experiments.

### Example 4: Synergistic effect of 4-OHT in combination with another anti-cancer agent

For determining the synergistic effect SE₁, breast cancer cell lines T-47D, ZR-75-1, MDAMB453 were cultured as described above Then, cells were either treated with 4-OHT (OHT) at a concentration of 10 or 20 µM as control, or with the anti-cancer agent Enzalutamide (EN) at a concentration of 5 or 10 µM, Endoxifen (ETM) at a concentration of 25 nM, Fulvestrant (FUL) at a concentration of 1.0 µM, or Exemestane (EXE) at a concentration of 0.1 µM alone, Palbociclib (Pab) at a concentration of 50 nM, or Docextaxel (Doc) at a concentration of 1.0 nM alone or in combination with 4-OHT.

As summarized in **Table 2**, simultaneous treatment of breast cancer cell lines with 4-OHT and the other anti-cancer agent resulted in a synergistic effect. Overall, best results are obtained regarding the cell line T-47D offering a Luminal A breast cancer type with expression of ER; PR and AR receptors. Results correspond to a least four independent experiments with an incubation time of 4 days

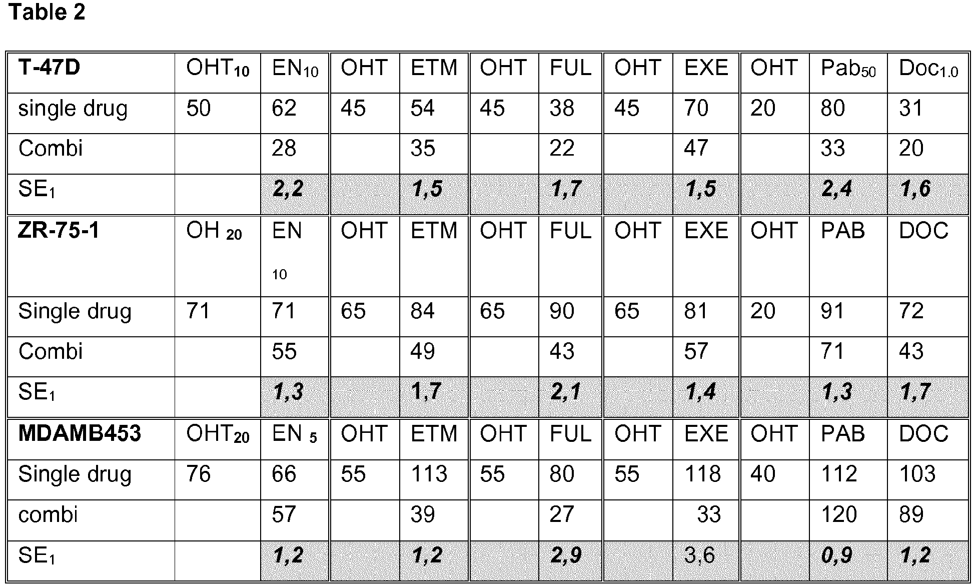

### Example 5: Combination of 4-OHT with androgen receptor inhibitors or androgen synthesis inhibitors Bicalutamide or Enzalutamide

For assessement of synergies between androgen receptor antagonists and androgen receptor synthesis inhibitors, human prostate cancer cell lines LNCaP and VCaP were used.

LNCaP (ATCC No.: CRL-1740™) is a human prostate cell line isolated from a distant lymph node of a prostate cancer patient (Horoszewicz et al. 1983). This cell line expresses a functional AR receptor. Due to a mutation in the AR and high levels of ER expression, LNCaP cells respond also to estrogen activation.
VCaP (ECACC cell line) is a cell line originally described by Korenchuk et al (2011) and represents a human prostate cancer cell line with high expression of the AR receptor (overexpression) and androgen sensitivity.
Both cell lines were cultured in the corresponding cell culture medium as recommended in the guidelines (ATCC, DSMZ). Medium was changed depending on glucose consumption and cell density. At 60-75% confluence, cells were harvested by trypsin/EDTA (0.05% Trypsin, 0.02% EDTA, Sigma Aldrich). Cell number/ viability was determined by trypan blue staining either counting using a hemocytometer and/or Countess Imaging (Life technologies).

Prior to subculture onto 96-well multi-well plates, one vessel of cells (25 cm²) was starved in the corresponding medium lacking phenol red and replacing fetal calf serum (FCS) by charcoal stripped (CS) FCS (termed -/- medium, full medium is termed +/+ medium). Depending on the individual experimental design, only cells without preceding starvation (termed +/+) were used (details see legends to figures).

LNCaP cells were subcultered onto 96 wells MTP and treated with 4-OHT at a concentration of 10 µM, Bicalutamide at a concentration of 5 or 10 µM and Enzalutamide at a concentration of of 5 or 10µM as single compounds. Additionally, cells were treated simultaneously with 4-OHT in combination with Bicalutamide 10 µM and Enzalutamide 10µM with subculture onto MTP. The growth of the cells and growth inhibition were monitored at A 450 nm values including standard errors from WST-1 assay at day 5.

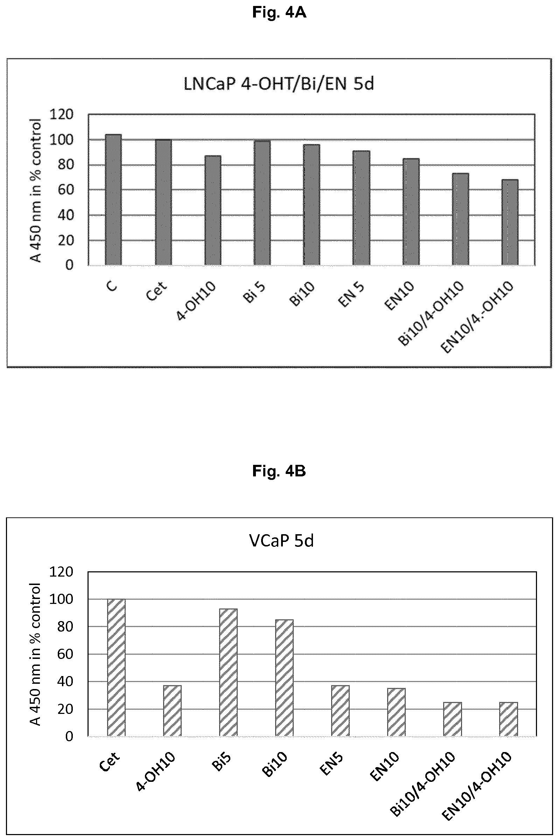

As can be seen in **Fig. 4A**, LNCaP cells are not affected by Bicalutamide or Enzalutamide alone and 4-OHT has only a slight effect. However, combination of 4-OHT with both drugs results in growth inhibition with a significant synergistic effect, wherein "C" denotes control and "Cet" solvent control (ethanol).

As illustrated in **Fig. 4B**, VCap cells react very sensitive on 4-OHT and Enzalutamide, while Bicalutamide is not showing a significant growth inhibitors effect. However, combination of Bicalutamide and 4-OHT as well as 4-OHT and enzalutamide showed a synergistic effect on growth of this human tumor cell line, wherein "Cet" solvent control (ethanol).

### Example 6: Combination of 4-OHT with androgen synthesis inhibitors Abiraterone

LNCaP cells were subcultered onto 96 wells MTP and treated with drugs 4-OHT at a concentration of 10 µM or Abiraterone at concentration of 10 µM as single compounds. Additionally, cells were treated simultaneously with 4-OHT in combination with Abiraterone. The growth of the cells and growth inhibition were monitored at A 450 nm values including standard errors from WST-1 assay at day 5.

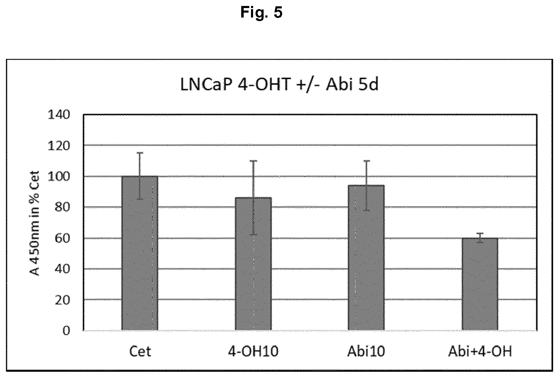

As can be seen in **Fig. 5**, LNCaP cells are not affected by Abiraterone and 4-OHT has only a slight effect. However, combination of 4-OHT with these drugs results in a significant synergistic effects and growth inhibition, wherein "Cet" solvent control (ethanol).

### Example 7: Synergistic effect of 4-OHT in combination with other anticancer agent

For assessing synergy, LNCaP and VCaP cells were cultured as described above.

Then, cells were treated either with 4-OHT, Enzalutamide at a concentration of 10 µM (EN10) or Abiraterone at a concentration of 10 µM (ABI) as single compounds or simultaneously with a combination of 4-OHT and Enzalutamide or 4-OHT and Abiraterone (Combi), respectively. Synergie (SE) was calculated as ratio A_{cod}/A_{combi} (SE₁), wherein A is the absorbance at 450 nm as % of solvent control, A_{cod} is the absorbance of "common drugs" like EN/ABI, and A_{combi} the absorbance when COD was applied in combination with 4-OHT. SE values correspond to the results of at least four independent experiments and are summarized in **Table 3**.

Obviously no remarkable differences were observed between S₁ values. SE-values above 1.0 offer a benefit by simultaneous treatment of 4-OHT and COD as compared to a single administration of EN/ABI, respectively.

**Table 3**

| Cell line | **4-OHT** | **EN10** | Combi | SE₁ | **ABI** | Combi | SE₁ |
|---|---|---|---|---|---|---|---|
| LNCaP | 78 | 84 | 58 | ***1,45*** | 96 | 90,57 | ***1,06*** |
| VCaP | 33 | 32 | 30 | ***1,06*** | 104 | 28,11 | ***3,7*** |

### General observation of results

Given the surprising finding that 4-OHT acts synergistically when the compound is combined with so distinct anti-cancer agents such as CKD4/CKD inhibitors, aromatase inhibitors, chemotherapeutics, androgen receptor inhibitors, androgen synthesis inhibitors and other anticancer agents according to Examples 1 to 7, it is demonstrated that 4-OHT and related compounds as disclosed herein surprisingly has an entirely new therapeutic effect different from classical anti-cancer mechanisms and independent from an anti-cancer agent to be combined with. The data rather demonstrate a general effectivity across anti-cancer treatment regimes and allows new therapeutic uses as disclosed herein.

## Claims

1. A combination for use in the therapy of cancer comprising:
(i) a first active compound being selected from compounds having the following formula I: 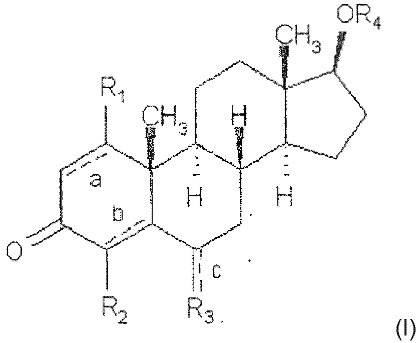
wherein a, b and c respectively denote, independently from each other, a single bond or a double bond, with the proviso that at least one of a, b and c represents a double bond, and with the proviso that if a is single bond and b is double bond R₂ is not H;
R₁ is hydrogen or C₁ to C₆ alkyl;
R₂ is OR5 or hydrogen, wherein R₅ is hydrogen or C₁ to C₆ straight chain or branched alkyl; R₃ is, in case of c being a single bond, hydrogen or C₁ to C₆ alkyl, or in case of c being a double bond, CHR₅, wherein R₅ is the same as defined before;
R₄ is hydrogen, C₁ to C₆ alkyl, phenyl unsubstituted or substituted by C₁ to C₆ alkyl, COR₆ acyl group, wherein R₆ is hydrogen; C₁ to C₁₂ straight chain or branched alkyl; phenyl or benzoyl respectively unsubstituted or substituted by C₁ to C₆ alkyl, or any group leading to hydroxyl upon biological metabolization or chemical deprotection; and salts thereof;
(ii) a second active anti-cancer compound different from compound (i),
and wherein compound (ii) is used under one or more of the following conditions:
(a) for a patient to be sensitized for said anti-cancer compound (ii), the patient being insensitive or resistant against therapy by said anti-cancer compound;
(b) at a dose and/or a dosage regimen of a known and conventionally used anti-cancer agent as second compound (ii), lower than the dose and/or dosage regimen conventionally used or admitted for said compound (ii) in the therapy of the same cancer;
(c) for a patient who, due to side effects caused by the second active anti-cancer compound (ii), would without the first active compound (i) be determined for treatment by an add-on therapy to counteract the side effects;
(d) for a patient who cannot be treated with said second active anti-cancer compound (ii) at a dose and/or a dosage regimen of a known and conventionally used anti-cancer agent as second compound (ii) conventionally used or admitted for said compound (ii) in the therapy of the same cancer due to side effects caused by said anti-cancer compound (ii), or for a patient who cannot be treated with said second active anti-cancer compound (ii) due to side effects caused by said anti-cancer compound (ii) at all.

2. A compound (i) of formula (I), defined as set forth in claim 1, for use in the treatment of a patient suffering from cancer, who previously has been treated with at least one other anti-cancer compound (ii) and has failed with such previous treatment.

3. The combination or compound (i) for use according to any of the preceding claims, wherein compound (i) is defined by a and c being a single bond, b being a double bond, and R₂ being OR5, OR5 being as defined in claim 1, preferably wherein compound (i) is 4-OHT or a salt or an analogue thereof.

4. The combination or compound (i) for use according to any of the preceding claims, wherein the second active anti-cancer compound (ii) is selected from the group consisting of a CDK4/CDK inhibitors; an aromatase inhibitor; an estrogen antagonist; an estrogen receptor modulator, a chemotherapeutic drug; an HER2/neu receptor antagonist; an androgen receptor inhibitor; an androgen synthesis inhibitor; an androgen synthesis antagonist; a GnRH receptor antagonist; a GnRH agonist; a microtubule inhibitor; an anthracenedione antineoplastic agent; steroids, specific antibody therapies; immune therapeutics.

5. The combination or compound (i) for use according to any of the preceding items, wherein the second active anti-cancer compound (ii) is selected from the group consisting of Palbociclib, Ribociclib, Abemaciclib; Exemestan, Anastrozole, Letrozole; Fulvestrant; Endoxifen, Tamoxifen, Raloxifene, Ospemifene, Toremifene, Docetaxel, Abraxane, Doxorubicin, or Cyclophsophamide; Trastuzumb; Bicalutamide, Flutamide, Nilutamide; Enzalutamide, Abiraterone, Apalutamide; Goserelin; Degarelix; Leuprolide; Cabazitaxel, Mitoxantrone.

6. The combination or compound (i) for use according to any of the preceding items, wherein the cancer is a cancer and/or metastasis thereof that is conventionally treated with the second active anti-cancer compound (ii) as defined in any of the preceding items.

7. The combination or compound (i) for use according to any of the preceding items, wherein the cancer is a breast cancer, an ovarian cancer, an endometrial cancer, a prostate cancer or a testis cancer, preferably wherein the cancer is a breast cancer or a prostate cancer, more preferably wherein the cancer is an advanced breast cancer or an advanced prostate cancer.

8. The combination or compound (i) for use according to any of the preceding items, wherein the cancer is AR-positive, preferably wherein the cancer is AR-positive with an AR-expression of at least 1%.

9. The combination for use according to any of the preceding items, wherein the first active compound (i) and the second active anti-cancer compound (ii) are administered to a patient simultaneously or in the order of firstly using or releasing the first active compound (i) and subsequently using or releasing the second active anti-cancer compound (ii), respectively, optionally in common or in separate dosage forms.

10. The compound (i) for use according to claim 2, wherein the cancer is either a breast cancer and the at least one other anti-cancer compound (ii) that has failed is an anti-estrogen, particularly an aromatase inhibitor and/or estrogen receptor antagonist, particularly fulvestrant and/or an estrogen receptor modulator, particularly tamoxifen or alternatively, the cancer is prostate cancer and the at least one other anti-cancer compound that has failed is a GnRH agonist and/or Docetaxel and/or an androgen synthesis inhibitor, particularly abiraterone, or bicalutamide and/or an androgen receptor antagonist, particularly as enzalutamide.

11. A combination comprising:
(i) a compound (i) of formula (I) as defined in any the preceding claims, and
(ii) an anti-cancer compound different from compound (i),
wherein said anti-cancer compound is selected from the group consisting of a CDK4/CDK inhibitor, preferably Palbociclib, Ribociclib, or Abemaciclib; Exemestan; Anastrozole; Letrozole; Fulvestrant; an estrogen receptor modulator, particularly Endoxifen, Tamoxifen, Raloxifene, Ospemifene, Toremifene; a chemotherapeutic drug, particularly Docetaxel, Abraxane, Doxorubicin, or Cyclophsophamide; Trastuzumb; Nilutamide; an androgen synthesis inhibitor, particularly Enzalutamide, Abiraterone, Apalutamide; an androgen synthesis antagonist, particularly Goserelin; a GnRH receptor antagonist, particularly Degarelix; a GnRH agonist, particularly Leuprolide; a microtubule inhibitor, particularly Cabazitaxel; an anthracenedione antineoplastic agent, particularly Mitoxantrone; immunotherapeutically active substance including specific tumor antibody and/or immune therapeutic agent, particularly monoclonal antibodies to treat cancer, chimeric antigen receptor (CAR) T-cell therapy, immune checkpoint inhibitors to treat cancer, cancer vaccines and/or non-specific cancer immunotherapies and adjuvants, particularly cytokines including interleukins and interferons.

12. A combination according to claim 11 for use in the therapy of cancer, preferably wherein the cancer is a cancer as defined in any of the preceding claims.

13. The combination for use according to any of claims 1, 3 to 10 or a combination according to claim 11, wherein the first active compound (i) and the second active anti-cancer compound (ii) are comprised in a common or in separate dosage forms, preferably wherein the combination is arranged in a package together with instructions providing descriptions in relation to any one of conditions (a) to (d), in particular descriptions relating to specific dose and/or dosage regimen.

14. A pharmaceutical formulation for use according to any of the preceding claims comprising the first active compound (i), and optionally the second active anti-cancer compound (ii).

15. The pharmaceutical formulation for use according to claim 14, wherein the formulation is for topical, transdermal, parenteral, intranasal, oral, rectal, intraocular administration and/or for instillation, preferably wherein the formulation is for topical administration.
